# FASCICULE DE BREVET EUROPEEN

(11) **EP 1 629 829 B1**
(45) Date de publication et mention de la délivrance du brevet: **25.01.2017**
(21) Numéro de dépôt: 05291490.0
(22) Date de dépôt: 11.07.2005
(51) Int. Cl.: A61K 8/85, A61K 8/87, A61Q 5/06, A61K 8/73

(54) **Procédé de coiffage mettant en oeuvre une composition cosmétique comprenant dans un milieu non gras un polyester sulfonique linéaire et polymère épaississant non-ionique**
Haarstylingverfahren auf Basis einer kosmetischen Zusammensetzung enthaltend ein sulfoniertes Polymer und ein nichtionisches Verdickungspolymer
Hairstyling method comprising the application of a cosmetic composition comprising a linear sulfonic polyester and a non-ionic thickening polymer

(30) Priorité: 29.07.2004 FR 0408398
(43) Date de publication de la demande: 01.03.2006
(73) Titulaire: L'Oréal, 75008 Paris (FR)
(72) Inventeur: Vrignaud, Sabine, 93210 La Plaine (FR); Laurent, Ludivine, 92400 Courbevoie (FR)
(74) Mandataire: Dossmann, Gérard

(56) Documents cités:
- EP-A- 1 400 234
- FR-A- 2 844 709
- FR-A- 2 853 528
- US-A1- 2002 189 030

## Description

La présente demande a pour objet une composition cosmétique comprenant, dans un milieu cosmétiquement acceptable non gras, au moins un polyester sulfonique linéaire dispersible dans l'eau et au moins un polymère épaississant non-ionique, ainsi que les procédés mettant en oeuvre cette composition. La présente demande concerne encore l'utilisation d'une composition cosmétique, comprenant, dans un milieu cosmétiquement acceptable non gras, au moins un polyester sulfonique linéaire dispersible dans l'eau et au moins un polymère épaississant non-ionique pour fixer la coiffure.

Les compositions cosmétiques pour la mise en forme et/ou le maintien de la coiffure peuvent être des compositions à pulvériser essentiellement constituées d'une solution le plus souvent alcoolique et d'un ou plusieurs composants, appelés composants fixants, qui sont généralement des résines polymères dont la fonction est de former des soudures entre les cheveux ou de gainer les cheveux. Ces composants fixants sont souvent formulés en mélange avec divers adjuvants cosmétiques.

Ces compositions cosmétiques sont généralement conditionnées soit dans un flacon pompe, soit dans un récipient aérosol approprié mis sous pression à l'aide d'un agent propulseur, le système aérosol contient alors d'une part une phase liquide (ou jus) et d'autre part d'un agent propulseur.

On peut également utiliser des compositions de coiffage sous forme de gels, de crèmes, de mousses.

Une fois appliqués sur les cheveux, les composés fixants doivent permettre la fixation de la chevelure.

Or, les polymères couramment utilisés en tant qu'agents fixants dans les compositions de coiffage ne permettent pas de conserver la mise en forme de la coiffure lorsque celle-ci est mise au contact de l'eau pendant une durée prolongée telle que par exemple une mise en contact avec la pluie, la transpiration, ou lors des bains : bains de mer ou bains en piscine...

De manière surprenante et avantageuse, la demanderesse a découvert que l'utilisation d'une composition cosmétique, comprenant, dans un milieu cosmétiquement acceptable, au moins un polyester sulfonique linéaire dispersible dans l'eau et au moins un polymère épaississant non-ionique permet de fixer et de mettre en forme les coiffures et aussi de conserver la mise en forme des coiffures lorsque celles-ci sont mises au contact de l'eau pendant une durée prolongée.

Au sens de la présente demande, on nomme ce phénomène de « résistance à l'eau ».

Par « durée prolongée » au sens de la présente demande, on entend une mise en contact avec l'eau d'une durée minimale d'une minute, de préférence 10 minutes, de manière 20 minutes.

Les compositions de l'invention permettent également d'obtenir un coiffage résistant à l'humidité de l'air.

Les compositions selon la présente invention permettent une bonne fixation et un bon maintien des cheveux, c'est-à-dire un effet coiffant qui persiste tout au long de la journée, voire plusieurs jours, qui présente une bonne résistance à l'eau, notamment une bonne résistance aux bains répétés, ces compositions présentent aussi l'avantage de s'éliminer au shampooing.

Ces compositions permettent aussi de conférer de bonnes propriétés cosmétiques aux cheveux.

La présente invention a pour objet une composition cosmétique, comprenant, dans un milieu cosmétiquement acceptable non gras, au moins un polyester sulfonique linéaire dispersible dans l'eau et au moins un polymère épaississant non-ionique.

Un autre objet de la présente invention consiste en un procédé pour la mise en forme ou le maintien de la coiffure dans lequel la composition cosmétique selon l'invention est mise en oeuvre.

Un troisième objet de l'invention concerne l'utilisation d'une composition cosmétique comprenant, dans un milieu cosmétiquement acceptable non gras, au moins un polyester sulfonique linéaire dispersible dans l'eau et au moins un polymère épaississant non-ionique en tant que composition coiffante pour la fixation et le maintien des cheveux en particulier lorsqu'ils sont mis au contact de l'eau pendant une durée prolongée c'est-à-dire en cas de pluie, de transpiration, lors de bains : tels que les bains de mer ou en piscine.

Selon ce troisième objet de l'invention, l'utilisation de la composition permet l'obtention d'une coiffure (mise en forme des cheveux) résistant à l'eau.

La fixation ou le maintien des cheveux est conservé après une mise au contact des cheveux avec l'eau pendant une durée prolongée.

La composition cosmétique utilisée selon l'invention peut se présenter sous toutes les formes : lotions, sprays, mousses, gels, crèmes, pâtes.

D'autres objets, caractéristiques, aspects et avantages de l'invention apparaîtront encore plus clairement à la lecture de la description et des exemples qui suivent.

Par « composition cosmétique coiffante » au sens de la présente demande, on entend une composition pour la mise en forme ou le maintien de la coiffure.

Le milieu cosmétiquement acceptable utilisé dans les compositions selon la présente demande est un milieu non gras aqueux, hydro-alcoolique ou alcoolique contenant éventuellement au moins un solvant organique additionnel.

L'alcool utilisé dans les compositions selon la présente invention est alors un alcanol monohydroxylé choisi parmi les alcools inférieurs en C₁-C₄ comme l'éthanol, l'isopropanol, le tertiobutanol, le n-butanol, de préférence l'alcool utilisé est l'éthanol.

La concentration en alcool dans les compositions selon la présente invention est comprise entre 0 et 50%, de préférence entre 0 et 10% et de manière encore plus préférée entre 0 et 5% en poids par rapport au poids total de la composition.

À titre de solvant organique additionnel utilisable dans les compositions selon la présente invention, on compte les polyols comme le propylèneglycol, les éthers de polyols et leurs mélanges.

La concentration en solvant, organique additionnel dans les compositions selon la présente invention est comprise entre 0 et 30%, de préférence entre 0 et 20% en poids par rapport au poids total de la composition.

Par « milieu non gras » au sens de la présente demande, on entend que la quantité en poids de corps gras présent(s) dans le milieu est compris entre 0 et 10%, de préférence entre 0 et 5% par rapport au poids total de la composition.

Les corps gras utilisables dans les compositions selon la présente demande sont les corps gras siliconés ou non siliconés en particulier ce sont des huiles minérales ou végétales, siliconées ou non-siliconées, des cires ou leurs mélanges.

Au sens de la présente demande, on considère également comme corps gras tout composé liposoluble, en particulier tout polymère liposoluble compris dans la phase grasse.

Les huiles de silicone utilisables dans les compositions selon l'invention sont des polyméthylsiloxanes volatiles ou non, à chaîne siliconée linéaire ou cyclique, liquides ou pâteuse à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, les polyméthyl-phénylsiloxanes ; et leurs mélanges.

Les corps gras non siliconés utilisables dans les compositions selon la présente invention sont toutes les huiles, cires, résines non siliconées organiques ou minérales, naturelles ou synthétiques.

Une huile, au sens de la présente invention, est un composé lipophile, liquide à température ambiante (environ 25 °C), à changement d'état solide/liquide réversible. Les huiles animales et végétales comprennent comme constituants essentiels des triesters du propane-1,2,3-triol.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïques ou octanoïques ou encore, par exemple les huiles de tournesol, de maïs, de soja, de courge, de pépins de raisin, de sésame, de noisette, d'abricot, de macadamia, d'arana, de tournesol, de ricin, d'avocat, les triglycérides des acides caprylique/caprique comme par exemple ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R⁶COOR⁷ et R⁶OR⁷ dans laquelle R⁶ représente le reste d'un acide gras comportant de 8 à 29 atomes de carbone, et R⁷ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononaoate de diéthylèneglycol ; et les esters de pendaérythritol comme le tétraisostéarate de pentaérythrityle ;

- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras fluides ayant de 8 à 26 atomes de carbone, comme l'octyldodécanol, le 2-butyloctanol, l'alcool oléique, l'alcool linoléique ou l'alcool linolénique ;
- les alcools gras alcoxylés et notamment éthoxylés tels que l'oleth-12 ;
- les huiles fluorées partiellement hydrocarbonées comme celles décrites dans le document JP-A-2 295912. Comme huiles fluorées, on peut citer aussi le perfluorométhylcyclopentane et le perfluoro-1,3- diméthylcyclohexane, par exemple vendus sous les dénominations de « FLUTEC PC1® » et « FLUTEC PC3® » par la Société BNFL Fluorochemicals ; le perfluoro-1,2-diméthylcyclobutane ; les perfluoralcanes tels que le dodécafluoropentane et le tétradécafluorohexane, par exemple vendus sous les dénominations de « PF 5050® » et « PF 5060® » par la Société 3M, ou encore le bromoperfluorooctyle par exemple vendu sous la dénomination « FORALKYL® » par la Société Atochem ; le nanofluorométhoxybutane par exemple vendu sous la dénomination « MSX 4518® » par la Société 3M et le nanofluoroéthoxyisobutane ; les dérivés de perfluoromorpholine, tels que la 4-trifluorométhyl perfluoromorpholine par exemple vendue sous la dénomination « PF 5052® » par la Société 3M.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/on alcool.

Une cire, au sens de la présente invention, est un composé lipophile, solide à température ambiante (environ 25 °C), à changement d'état solide/liquide réversible, ayant une température de fusion supérieure à environ 40°C et pouvant aller jusqu'à 200°C, et présentant à l'état solide une organisation cristalline anisotrope. Les cires animales et végétales comprennent comme constituants essentiels des esters d'acides carboxyliques et d'alcools à longues chaînes. D'une manière générale, la taille des cristaux de la cire est telle que les cristaux diffractent et/ou diffusent la lumière, conférant à la composition qui les comprend un aspect trouble plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange, détectable microscopiquement et macroscopiquement (opalescence).

A titre de cires utilisables dans la présente invention, on peut citer les cires d'origine animale telles que la cire d'abeille, le spermaceti, la cire de lanoline et les dérivés de lanoline ; les cires végétales telles que les cires de tournesol, de riz, de pomme, la cire de Carnauba, de Candellila, d'Ouricury, du Japon, le beurre de cacao ou les cires de fibres de liège ou de canne à sucre ; les cires minérales, par exemple, de paraffine, de vaseline, de lignite ou les cires microcristallines, la cérésine ou l'ozokérite ; les cires synthétiques telles que les cires de polyéthylènes, les cires de Fischer-Tropsch, et leurs mélanges.

La composition selon l'invention comprend un polyester sulfonique linéaire dispersible dans l'eau.

Par polyester sulfonique linéaire hydrodispersible, on entend tout polyester sulfonique présentant une aptitude à former une dispersion, c'est-à-dire un système biphasique où la première phase est formée de particules finement divisées et distribuées uniformément dans la seconde phase qui est la phase continue.

Par polyester sulfonique, on entend des copolyesters obtenus par polycondensation d'au moins un acide dicarboxylique ou d'un de ses esters, d'au moins un diol et d'au moins un composé difonctionnel sulfoaryldicarboxylique substitué sur le noyau aromatique par un groupe -SO₃M dans lequel M représente un atome d'hydrogène ou un ion métallique tel que Na⁺, Li⁺ ou K⁺.

Les polyesters sulfoniques linéaires dispersibles dans l'eau présentent généralement une masse moléculaire moyenne en poids comprise entre environ 1 000 et 60 000, et de préférence de 4 000 à 20 000, telle que déterminée par chromatographie par perméation de gel (ou GPC).

La température de transition vitreuse (Tg) de ces polyesters sulfoniques est généralement comprise dans l'intervalle allant de 10 °C à 100 °C. De préférence, la Tg du ou des polyesters utilisés est supérieure ou égale à 50°C.

La température de transition vitreuse (Tg) est mesurée par analyse enthalpique différentielle (DSC, *differential scanning calorimetry*) selon la norme ASTM D3418-97.

Ils sont décrits plus en détail dans les demandes de brevet US 3 734 874, US 3 779 993, US 4 119 680, US 4 300 580, US 4 973 656, US 5 660 816, US 5 662 893, et US 5 674 479.

Les polyesters sulfoniques utilisés de préférence dans l'invention comprennent au moins des motifs dérivés d'acide isophtalique, de sel d'acide sulfoaryle-dicarboxylique et de diéthylèneglycol, et plus particulièrement les polyesters sulfoniques utilisés dans l'invention sont obtenus à partir d'acide isophtalique, de sel de sodium de l'acide sulfoisophtalique, de diéthylèneglycol et de 1,4-cyclohexanediméthanol.

A titre d'exemples de polyester sulfonique, on peut notamment citer ceux connus sous le nom INCI Diglycol/CHDM/Isophtalates/SIP, et vendus sous les dénominations commerciales"Eastman AQ polymer" (AQ35S, AQ38S, AQ55S, AQ48 Ultra) par la société Eastman Chemical.

De préférence, la Tg du ou des polyesters utilisés est supérieure ou égale à 50°C.

La concentration en polyester(s) sulfonique(s) linéaire(s) dispersible(s) dans l'eau utilisé(s) dans les compositions selon la présente invention est comprise entre 0,1 et 40%, de préférence entre 1 et 30% et de manière encore plus préférée entre 5 et 25% en poids par rapport au poids total de la composition.

La composition selon l'invention comprend au moins un polymère épaississant non-ionique.

Les polymères épaississants de type non ionique utilisables selon l'invention encore appelés « agents d'ajustement de la rhéologie » sont choisis parmi les épaississants cellulosiques tels que les hydroxyméthylcellulose, hydroxyéthycellulose, hydroxypropylcellulose, hydroxypropylméthylcellulose, la gomme de guar, la gomme de guar hydroxypropylée, et la gomme de scléroglucane.

Les polymères épaississants de type non ionique sont ainsi choisis de préférence parmi les polymères associatifs non-ioniques ci-dessous :
- (1) les celluloses modifiées par des groupements comportant au moins une chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
   - celles modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL.
- (2) les hydroxypropylguars modifiés par des groupements comportant au moins une chaîne grasse tel que le produit ESAFLOR HM 22® (chaîne alkyle en C₂₂) vendu par la société LAMBERTI, les produits RE210-18® (chaîne alkyle en C₁₄) et RE205-1® (chaîne alkyle en C₂₀) vendus par la société RHONE POULENC.

La concentration en polymère(s) épaississant(s) non-ionique(s) utilisé(s) dans les compositions selon la présente invention est comprise entre 0,01 et 10%, de préférence entre 0,05 et 5% et de manière encore plus préférée entre 0,1 et 2% en poids par rapport au poids total de la composition.

Avantageusement, le rapport polyester sulfonique linéaire dispersible dans l'eau / polymère épaississant non-ionique est compris entre 2 et 50%, de préférence entre 2,5 et 20% et encore plus préférentiellement entre 3 et 15% bornes comprises.

Les compositions selon la présente demande peuvent également contenir un ou plusieurs adjuvants cosmétiques additionnels tels que ceux mentionnés ci-dessous.

Tous les polymères fixants anioniques, cationiques, amphotères, non ioniques et leurs mélanges utilisés dans la technique peuvent être utilisés dans les compositions selon la présente demande.

Les polymères fixants additionnels peuvent être solubles dans le milieu cosmétiquement acceptable ou insolubles dans ce même milieu et utilisés dans ce cas sous forme de dispersions de particules solides ou liquides de polymère (latex ou pseudolatex).

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono ou diacides carboxyliques insaturés tels que ceux répondant à la formule : 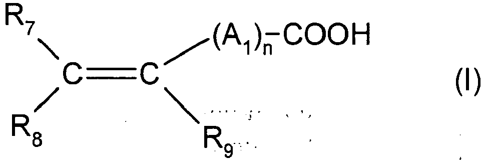 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel qu'oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

De préférence, les polymères fixants anioniques sont choisis parmi les homopolymères ou copolymères d'acide acrylique et méthacrylique ou leurs sels, les copolymères d'acide crotonique, les copolymères d'acides ou d'anhydrides carboxyliques monoinsaturés en C₄-C₈, les polyacrylamides à groupements carboxylates, les homopolymères ou copolymères à groupes sulfoniques, les polyuréthanes anioniques, et les polymères siliconés greffés anioniques.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL^{®} E ou K par la société ALLIED COLLOID et ULTRAHOLD^{®} par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques ;
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevet luxembourgeois n^{os} 75370 et 75371 ou proposés sous la dénomination QUADRAMER par la société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE^{®} LM et les terpolymères acide méthacrylique/acrylate d'éthyle/acrylate de tertiobutyle tels que le produit commercialisé sous la dénomination LUVIMER^{®} 100 P par la société BASF ;
   On peut aussi citer les copolymères acide méthacrylique/ acide acrylique/acrylate d'éthyle/méthacrylate de méthyle en dispersion aqueuse, commercialisé sous la dénomination AMERHOLD® DR 25 par la société AMERCHOL ;
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant, au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch ;
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n^{os} 2 047 398, 2 723 248, 2 102 113, le brevet GB n° 839 805. Des produits commerciaux sont notamment ceux vendus sous les dénominations GANTREZ^{®} AN ou ES par la société ISP ;
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.
   Ces polymères sont par exemple décrits dans les brevets français n^{os} 2 350 384 et 2 357 241 de la demanderesse ;
E) Les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone ;
- les sels de l'acide polystyrène-sulfonique tels que les sels de sodium vendus par exemple sous la dénomination Flexan^{®} 500 et Flexan^{®} 130 par National Starch. Ces composés sont décrits dans le brevet FR 2 198 719 ;
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par HENKEL.
Comme autre polymère fixant anionique utilisable selon l'invention, on peut citer le polymère anionique séquencé branché vendu sous la dénomination Fixate G-100 par la société NOVEON.

Selon l'invention, les polymères fixants anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD^{®} STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ^{®} par la société ISP, les copolymères d'acide méthacrylique et de méthacrylate de méthyle vendus sous la dénomination EUDRAGIT^{®} L par la société ROHM PHARMA, les copolymères d'acide méthacrylique et d'acrylate d'éthyle vendus sous la dénomination LUVIMER^{®} MAEX ou MAE par la société BASF et les copolymères acétate de vinyle/acide crotonique vendus sous la dénomination LUVISET CA 66 par la société BASF et les copolymères acétate de vinyle/acide crotonique greffés par du polyéthylèneglycol vendus sous la dénomination ARISTOFLEX^{®} A par la société BASF, le polymère vendu sous la dénomination Fixate G-100 par la société NOVEON.

Parmi les polymères fixants anioniques cités ci-dessus, on préfère plus particulièrement utiliser dans le cadre de la présente invention, les copolymères méthylvinyléther/anhydride maléique monoestérifiés vendus sous la dénomination GANTREZ^{®} ES 425 par la société ISP, les terpolymères acide acrylique/acrylate d'éthyle/N-tertiobutylacrylamide vendus sous la dénomination ULTRAHOLD^{®} STRONG par la société BASF, les copolymères d'acide méthacrylique et de méthacrylate de méthyle vendus sous la dénomination EUDRAGIT^{®} L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER^{®} MAEX OU MAE par la société BASF, les terpolymères vinylpyrrolidone/acide acrylique/méthacrylate de lauryle vendus sous la dénomination ACRYLIDONE^{®} LM par la société ISP, le polymère vendu sous la dénomination Fixate G-100 par la société NOVEON.

Les polymères filmogènes fixants cationiques utilisables selon la présente invention sont de préférence choisis parmi les polymères comportant des groupements amines primaires, secondaires, tertiaires et/ou quaternaires faisant partie de la chaîne polymère ou directement reliés à celle-ci, et ayant un poids moléculaire compris entre 500 et environ 5 000 000 et de préférence entre 1 000 et 3 000 000.

De préférence, les polymères fixants cationiques sont choisis parmi les homopolymères ou copolymères d'esters ou d'amides acryliques ou méthacryliques à fonctions aminées, les polysaccharides cationiques, les copolymères quaternaires de vinylpyrrolidone et de vinylimidazole, et les chitosanes.

Parmi ces polymères, on peut citer plus particulièrement les polymères cationiques suivants :
(1) les homopolymères ou copolymères dérivés d'esters ou d'amides acryliques ou méthacryliques et comportant au moins un des motifs de formules suivantes : 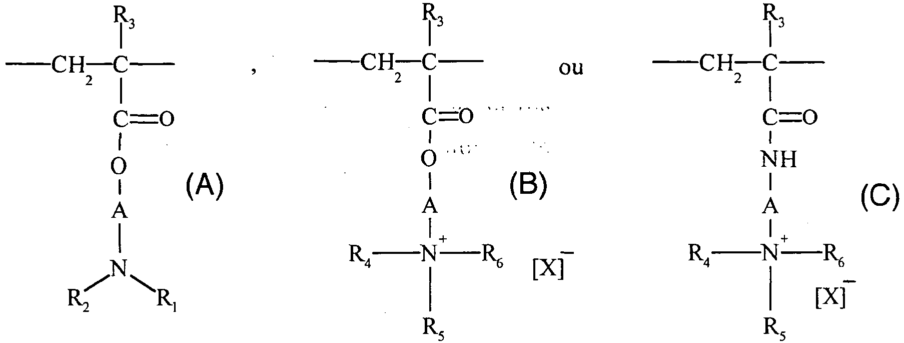 dans lesquelles:
   R₃ désigne un atome d'hydrogène ou un radical CH₃;
   A est un groupe alkyle linéaire ou ramifié comportant de 1 à 6 atomes de carbone ou un groupe hydroxyalkyle comportant de 1 à 4 atomes de carbone ;
   R₄, R₅, R₆, identiques ou différents, représentent un groupe alkyle ayant de 1 à 18 atomes de carbone ou un radical benzyle;
   R₁ et R₂, identiques ou différents, représentent chacun un atome hydrogène ou un groupe alkyle ayant de 1 à 6 atomes de carbone;
   X désigne un anion méthosulfate ou un halogénure tel que chlorure ou bromure.

   Les copolymères de la famille (1) contiennent en outre un ou plusieurs motifs dérivant de comonomères pouvant être choisis dans la famille des acrylamides, méthacrylamides, diacétones-acrylamides, acrylamides et méthacrylamides substitués sur l'azote par des groupes alkyles inférieurs (C1-C4), des groupes dérivés des acides acryliques ou méthacryliques ou leurs esters, des vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisé au sulfate de diméthyle ou avec un halogénure de méthyle, tels que celui vendu sous la dénomination HERCOFLOC® par la société HERCULES,
   - les copolymères d'acrylamide et de chlorure de méthacryloyloxy-éthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxy-éthyltriméthylammonium tel que celui vendu sous la dénomination RETEN par la société HERCULES,
   - les copolymères vinylpyrrolidone/acrylate ou méthacrylate de dialkylaminoalkyle quaternisés ou non, tels que les produits vendus sous la dénomination "GAFQUAT® " par la société ISP comme par exemple "GAFQUAT® 734" ou "GAFQUAT® 755" ou bien les produits dénommés "COPOLYMER® 845, 958 et 937". Ces polymères sont décrits en détail dans les brevets français 2.077.143 et 2.393.573,
   - les polymères à chaîne grasse et à motif vinylpyrrolidone, tels que les produits vendus sous la dénomination Stylèze W20 et Stylèze W10 par la société ISP,
   - les terpolymères méthacrylate de diméthylaminoéthyle/vinylcaprolactame/vinylpyrrolidone tel que le produit commercialisé sous la dénomination GAFFIX VC 713 par la société ISP, et
   - et les copolymères vinylpyrrolidone/méthacrylamide de diméthyl-aminopropyle quaternisés tels que les produits commercialisés sous la dénomination "GAFQUAT® HS 100" par la société ISP.
(2) les polysaccharides cationiques, de préférence à ammonium quaternaire tels que ceux décrits dans les brevets américains 3.589.578 et 4.031.307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL.
(3) les copolymères quaternaires de vinylpyrrolidone et de vinylimidazole ;
(4) les chitosanes ou leurs sels; les sels utilisables sont en particulier les acétate, lactate, glutamate, gluconate ou le pyrrolidone-carboxylate de chitosane .
   Parmi ces composés, on peut citer le chitosane ayant un taux de désacétylation de 90,5% en poids vendu sous la dénomination KYTAN BRUT STANDARD par la société ABER TECHNOLOGIES, le pyrrolidone-carboxylate de chitosane commercialisé sous la dénomination KYTAMER® PC par la société AMERCHOL .
(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyloxyéthyl triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-α,β-dicarboxylique dont l'un des groupements carboxyliques a été amené à réagir avec une polyamine comportant un ou plusieurs groupements amines primaires ou secondaires.

De préférence, les polymères fixants amphotères sont choisis parmi les copolymères à motifs vinyliques acides et à motifs vinyliques basiques, les polyaminoamides réticulés et acylés, les polymères à motifs zwittérioniques, les polymères dérivés du chitosane, les copolymères alkyl(C₁-C₅)vinyléther/anhydride maléique modifiés, les polyuréthanes amphotères et les polymères siliconés greffés amphotères.

Les polymères fixants amphotères répondant à la définition donnée ci-dessus plus particulièrement préférés sont choisis parmi les polymères suivants :
(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkyl-méthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les composés dont les groupes alkyles comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique, ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butylaminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethyl-methacrylate copolymer, tels que les produits vendus sous la dénomination AMPHOMER^{®} ou LOVOCRYL^{®} 47 par la société NATIONAL STARCH.
(3) les polyaminoamides réticulés et acylés partiellement ou totalement dérivant de polyaminoamides de formule générale : 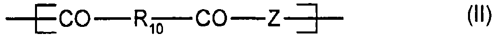 dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe 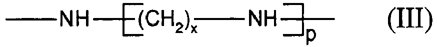 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (III) ci-dessus, dans lequel x=2 et p=1 et qui dérive de l'éthylène-diamine, ou le groupe dérivant de la pipérazine : 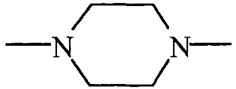
   c) dans les proportions de 0 à 20 % en moles, le groupe -NH-(CH₂)₆-NH- dérivant de l'hexaméthylènediamine,
   ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.
   Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
   Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule : 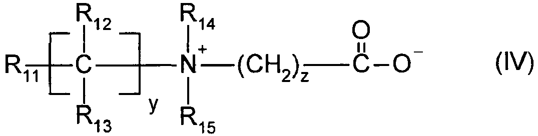 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthyl-carboxyméthylammonio-éthylméthacrylate de méthyle, tel que le produit vendu sous la dénomination DIAFORMER Z301 par la société SANDOZ.
(5) les polymères dérivés du chitosane comportant des motifs monomères répondant aux formules suivantes : 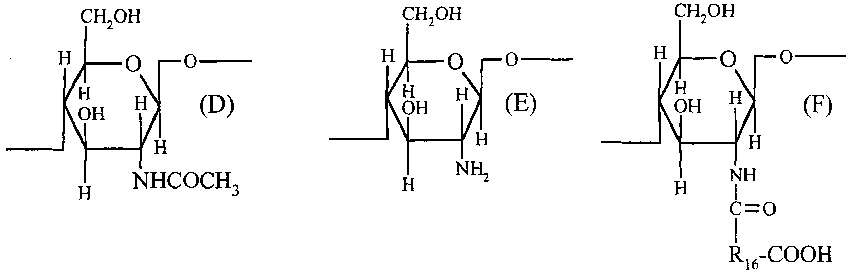 le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), R₁₆ représente un groupe de formule : 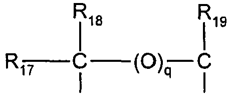
   dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères répondant à la formule générale (V) sont, par exemple, décrits dans le brevet français 1 400 366 : 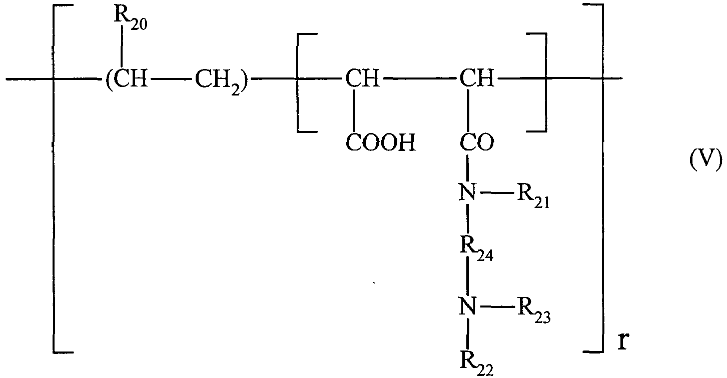 dans laquelle R₂₀ représente un atome d'hydrogène, un groupe CH₃O, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement -CH₂-CH₂- , -CH₂-CH₂-CH₂-, -CH₂-CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutyl-chitosane, vendus sous la dénomination « EVALSAN » par la société JAN DEKKER.
(8) Les polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (VI)

      où D désigne un groupe 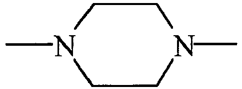 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (VI')

      où D désigne un groupe 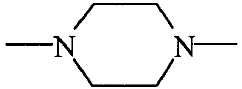 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyles et comportant un ou plusieurs atomes d'azote, l'atome d'azote étant substitué par une chaîne alkyle interrompue éventuellement par un atome d'oxygène et comportant obligatoirement une ou plusieurs fonctions carboxyles ou une ou plusieurs fonctions hydroxyles et bétaïnisées par réaction avec l'acide chloracétique ou du chloracétate de soude.
(9) les copolymères alkyl(C₁-C₅)vinyléther/anhydride maléique modifiés partiellement par semiamidification avec une N,N-dialkylaminoalkylamine telle que la N,N-diméthylaminopropylamine ou par semiestérification avec un N,N-dialkylaminoalcanol. Ces copolymères peuvent également comporter d'autres comonomères vinyliques tels que le vinylcaprolactame.

Parmi les polymères fixants amphotères cités ci-dessus les plus particulièrement préférés selon l'invention, on citera ceux de la famille (3) tels que les copolymères dont la dénomination CTFA est Octylacrylamide/acrylates/butylamino-ethylmethacrylate copolymer, tels que les produits vendus sous les dénominations AMPHOMER^{®}, AMPHOMER^{®} LV 71 ou LOVOCRYL^{®} 47 par la société NATIONAL STARCH et ceux de la famille (4) tels que les copolymères méthacrylate de méthyle/diméthyl-carboxyméthylammonio-éthyl-méthacrylate de méthyle vendu par exemple sous la dénomination DIAFORMER Z301 par la société SANDOZ.

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL^{®} AC-261 K et EUDRAGIT^{®} NE 30 D, par la société BASF sous la dénomination 8845, par la société HOECHST sous la dénomination APPRETAN^{®} N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH^{®} LDM 6911, MOWILITH^{®} DM 611 et MOWILITH^{®} LDM 6070 proposés par la société HOECHST, les produits RHODOPAS^{®} SD 215 et RHODOPAS^{®} DS 910 proposés par la société RHONE POULENC ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame différents des homopolymères de vinylpyrrolidone, tels que le polyvinylcaprolactame commercialisé sous la dénomination Luviskol^{®} PLUS par la société BASF ; et
- les copolymères de vinyllactame tels qu'un copolymère poly(vinylpyrrolidone/vinyllactame) vendu sous le nom commercial Luvitec^{®} VPC 55K65W par la société BASF, les copolymères poly(vinylpyrrolidone/acétate de vinyle) comme ceux commercialisés sous la dénomination PVPVA^{®} S630L par la société ISP, Luviskol^{®} VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol^{®} VAP 343 par la société BASF.

Les groupes alkyles des polymères non ioniques mentionnés ci-dessus ont, de préférence, de 1 à 6 atomes de carbone.

Selon l'invention, on peut également utiliser des polymères fixants de type siliconés greffés, comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0 582 152 et WO 93/23009 et les brevets US 4,693,935, US 4,728,571 et US 4,972,037.

Ces polymères peuvent être amphotères, anioniques ou non ioniques, et ils sont de préférence anioniques ou non ioniques.

De tels polymères sont, par exemple, les copolymères susceptibles d'être obtenus par polymérisation radicalaire à partir du mélange de monomères formé :
a) de 50 à 90 % en poids d'acrylate de tertiobutyle,
b) de 0 à 40 % en poids d'acide acrylique,
c) de 5 à 40 % en poids d'un macromère siliconé de formule 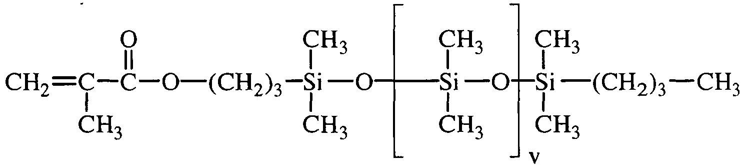 où v est un nombre allant de 5 à 700, les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type poly(acide (méth)acrylique) et du type poly((méth)acrylate d'alkyle), et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Comme autre type de polymères fixants siliconés, on peut citer le produit Luviflex^{®} Silk commercialisé par la société BASF.

On peut également utiliser comme polymères fixants, des polyuréthanes fonctionnalisés ou non, siliconés ou non, cationiques, non-ioniques, anioniques ou amphotères, ou leurs mélanges.

Les polyuréthanes particulièrement visés par la présente invention sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et EP 0 619 111 de la société National Starch.

Comme polyuréthanes convenant particulièrement bien dans la présente invention, on peut citer les produits commercialisés sous les dénominations LUVISET PUR^{®} et LUVISET^{®} Si PUR par la société BASF.

Lorsque la composition selon la présente invention comprend un ou plusieurs polymère(s) fixant(s) additionnels, leur concentration est comprise entre 0,1 et 20%, de préférence entre 0,5 et 10% en poids par rapport au poids total de la composition.

Les compositions selon la présente demande peuvent également contenir en tant qu'adjuvant cosmétique additionnel au moins un polymère épaississant additionnel encore appelé "agent d'ajustement de la rhéologie", ce polymère épaississant additionnel nommé polymère co-épaississant au sens de la présente invention est choisi parmi les polymères épaississants cationiques, anioniques amphotères.

La concentration en polymère(s) épaississant(s) additionnel(s) est comprise entre 0,01 et 20%, de préférence entre 0,05 et 10% en poids par rapport au poids total de la composition

La composition coiffante selon l'invention peut contenir en outre au moins un additif choisi parmi les agents tensio-actifs non-ioniques, anioniques, cationiques et amphotères, les polymères non-ioniques, anioniques, cationiques et amphotères additionnels autres que les polymères fixants utilisés dans les compositions selon l'invention, les céramides et pseudo-céramides, les vitamines et pro-vitamines dont le panthénol, les filtres solaires hydrosolubles et liposolubles, siliconés ou non siliconés, les charges et particules solides telles que par exemple les pigments minéraux et organiques, colorés ou non colorés, les agents nacrants et opacifiants, les paillettes, particules actives, les colorants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les agents de pénétration, les parfums et les agents conservateurs.

L'homme de métier veillera à choisir les éventuels additifs et leur quantité de manière à ce qu'ils ne nuisent pas aux propriétés des compositions selon la présente invention.

Ces additifs sont présents dans la composition selon l'invention en une quantité allant de 0 à 20 % en poids par rapport au poids total de la composition.

L'exemple qui suit illustre la présente invention et ne doit en aucune manière être considéré comme limitant l'invention.

### EXEMPLE

On prépare la formulation A suivante :

| Formulation A | en % de matière active |
|---|---|
| DIGLYCOL/CHDM/ISOPHTHALATES/SIP COPOLYMER (EASTMAN AQ 55 S - EASTMAN) | 20 |
| HYDROXYPROPYL GUAR (JAGUAR HP 105 - RHODIA) | 0,5 |
| DIMETHICONE (huile de silicone) | 1 |
| STEARYL ALCOHOL | 8 |
| EAU DESIONISEE | QS 100 |
| Parfum et ingrédients | QSP |

EASTMANN AQ 55 S commercialisé par EASTMANN est un copolymère de diéthylèneglycol/1,4-cyclohexanediméthanol/isophtalate/ sulfoisophtalate, c'est un polyester sulfonique linéaire dispersible dans l'eau tel que défini selon la présente demande.

HYDROXYPROPYL GUAR (JAGUAR HP 105 - RHODIA) est un polymère épaississant non ionique tel que défini selon la présente demande.

### Protocole opératoire :

- Sur mèche de cheveux naturels de 2,7g et de longueur 27 cm, appliquer 2 g de la formule à tester.
- Enrouler la mèche traitée autour d'un bigoudi de diamètre 1 cm, pour lui donner une forme.
- Laisser sécher le produit à l'air, puis défaire délicatement la mèche du bigoudi.
- Les mèches ainsi mises en forme sont ensuite immergées dans un bain d'eau salée (3% NaCl) de volume 8 litres à température ambiante, sous agitation magnétique à 100 tours/minute.
- Mesurer la longueur de la mèche au cours du temps afin d'évaluer le maintien de la mise en forme.

### Mesures du maintien de la mise en forme :

Maintien de la mise en forme en % : (Lᵢ - L)/(Lᵢ - L₀)* 100
L : Longueur de la mèche bouclée au temps t
L₀ : Longueur de la mèche bouclée après mise en forme et retrait du bigoudi
Lᵢ : Longueur de la mèche avant mise en forme sur bigoudi

| Temps d'immersion | **Maintien de la mise en forme en %** |
|---|---|
| | A |
| **0** | **100** |
| **35 secondes** | **100** |
| **40 secondes** | **100** |
| **2 minutes** | **100** |
| **5 minutes** | **100** |
| **10 minutes** | **100** |

## Revendications

1. Procédé pour la mise en forme ou le maintien de la coiffure dans lequel une composition cosmétique comprenant, dans un milieu cosmétiquement acceptable non gras dont la quantité en poids de corps gras est comprise entre 0 et 10%, de préférence entre 0 et 5%, par rapport au poids total de la composition, au moins un polyester sulfonique linéaire dispersible dans l'eau et au moins un polymère épaississant non-ionique, est mise en oeuvre, ledit au moins un polyester sulfonique linéaire dispersible dans l'eau étant un polycondensat d'au moins un acide dicarboxylique ou d'un de ses esters, d'au moins un diol et d'au moins un composé difonctionnel sulfoaryldicarboxylique substitué sur le noyau aromatique par un groupe -SO₃M dans lequel M représente un atome d'hydrogène ou un ion métallique tel que Na+, Li+ ou K+, ledit au moins un polymère épaississant non-ionique étant choisi parmi les épaississants cellulosiques, la gomme de guar, la gomme de guar hydroxypropylée et la gomme de scléroglucane.

2. Procédé selon la revendication 1, **caractérisé en ce que** le polyester sulfonique linéaire dispersible dans l'eau est obtenu à partir d'acide isophtalique, de sel de sodium de l'acide sulfoisophtalique, de diéthylèneglycol et de 1,4-cyclohexaneméthanol.

3. Procédé selon l'une quelconque des revendications précédentes, **caractérisé en ce que** la Tg du ou des polyester(s) sulfonique(s) linéaire(s) dispersible(s)dans l'eau utilisés est supérieure ou égale à 50°C.

4. Procédé selon l'une quelconque des revendications précédentes, **caractérisé en ce que** la concentration en polyester(s) sulfonique(s) linéaire(s) dispersible(s) dans l'eau est comprise entre 0,1 et 40%, de préférence entre 1 et 30% et de manière encore plus préférée entre 5 et 25% en poids par rapport au poids total de la composition.

5. Procédé selon l'une quelconque des revendications précédentes, **caractérisé en ce que** la composition comprend au moins un polymère épaississant non-ionique choisi parmi : les celluloses modifiées par des groupements comportant au moins une chaîne grasse ; les hydroxypropylguars modifiés par des groupements comportant au moins une chaîne grasse.

6. Procédé selon l'une quelconque des revendications précédentes, **caractérisé en ce que** la concentration en polymère(s) épaississant(s) non ionique(s) est comprise entre 0,01 et 10%, de préférence entre 0,05 et 5% et de manière encore plus préférée entre 0,1 et 2% en poids par rapport au poids total de la composition.

7. Procédé selon l'une quelconque des revendications précédentes, **caractérisé en ce que** le rapport polyester sulfonique linéaire dispersible dans l'eau / polymère(s) épaississant(s) non ionique est compris entre 2 et 50%, de préférence entre 2,5 et 20% et encore plus préférentiellement entre 3 et 15% bornes comprises.

8. Procédé selon l'une quelconque des revendications précédentes, **caractérisé en ce que** la composition comprend un polymère fixant choisi parmi les polymères fixants anioniques, cationiques, amphotères, non-ioniques, ou leurs mélanges.

9. Procédé selon l'une quelconque des revendications précédentes, **caractérisé en ce que** le polymère fixant cationique est choisi parmi les homopolymères ou copolymères d'esters ou d'amides acryliques ou méthacryliques à fonctions aminées, les polysaccharides cationiques, les copolymères quaternaires de vinylpyrrolidone et de vinylimidazole, et les chitosanes.

10. Procédé selon la revendication 8, **caractérisé en ce que** le polymère fixant anionique est choisi parmi les homopolymères ou copolymères d'acide acrylique et méthacrylique ou leurs sels, les copolymères d'acide crotonique, les copolymères d'acides ou d'anhydrides carboxyliques monoinsaturés en C₄-C₈, les polyacrylamides à groupements carboxylates, les homopolymères ou copolymères à groupes sulfoniques, les polyuréthanes anioniques, et les polymères siliconés greffés anioniques.

11. Procédé selon la revendication 8, **caractérisé en ce que** le polymère fixant amphotère est choisi parmi les copolymères à motifs vinyliques acides et à motifs vinyliques basiques, les polyaminoamides réticulés et acylés, les polymères à motifs zwittérioniques, les polymères dérivés du chitosane, les copolymères alkyl(C₁-C₅)vinyléther/anhydride maléique modifiés, les polyuréthanes amphotères et les polymères siliconés greffés amphotères.

12. Procédé selon la revendication 8, **caractérisé en ce que** le polymère fixant non ionique est choisi parmi les polyalkyloxazolines, les homopolymères et copolymères d'acétate de vinyle, les homopolymères et copolymères d'esters acryliques, les copolymères d'acrylonitrile, les homopolymères et copolymères de styrène, les polyamides, les homopolymères de vinyllactame différents des homopolymères de vinylpyrrolidone, les copolymères de vinyllactame, les polyuréthanes non ioniques, et les polymères siliconés greffés non ioniques.

13. Procédé selon l'une quelconque des revendications 8 à 12, **caractérisé en ce que** la concentration en polymère(s) fixant(s) est comprise entre 0,1 et 20%, de préférence entre 0,5 et 10% en poids par rapport au poids total de la composition.

14. Procédé selon l'une quelconque des revendications précédentes, **caractérisé en ce que** la composition comprend un polymère épaississant additionnel cationique anionique ou amphotère.

15. Procédé selon la revendication précédente, **caractérisé en ce que** la concentration en polymère épaississant additionnel est comprise entre 0,01 et 20%, de préférence entre 0,05 et 10% en poids par rapport au poids total de la composition.

16. Procédé selon l'une quelconque des revendications précédentes, **caractérisé en ce que** la composition contient au moins un additif choisi parmi les agents tensio-actifs non-ioniques, anioniques, cationiques et amphotères, les polymères non-ioniques, anioniques, cationiques et amphotères additionnels, les céramides et pseudo-céramides, les vitamines et pro-vitamines dont le panthénol, les filtres solaires hydrosolubles et liposolubles, siliconés ou non siliconés, les charges et particules solides telles que par exemple les pigments minéraux et organiques, colorés ou non colorés, les agents nacrants et opacifiants, les paillettes, particules actives, les colorants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les agents de pénétration, les parfums et les agents conservateurs.

17. Utilisation pour la fixation et le maintien des cheveux d'une composition comprenant, dans un milieu cosmétiquement acceptable non gras dont la quantité en poids de corps gras est comprise entre 0 et 10%, de préférence entre 0 et 5%, par rapport au poids total de la composition, au moins un polyester sulfonique linéaire dispersible dans l'eau et au moins un polymère épaississant non-ionique, ledit au moins un polyester sulfonique linéaire dispersible dans l'eau étant un polycondensat d'au moins un acide dicarboxylique ou d'un de ses esters, d'au moins un diol et d'au moins un composé difonctionnel sulfoaryldicarboxylique substitué sur le noyau aromatique par un groupe -SO₃M dans lequel M représente un atome d'hydrogène ou un ion métallique tel que Na+, Li+ ou K+, ledit au moins un polymère épaississant non-ionique étant choisi parmi les épaississants cellulosiques, la gomme de guar, la gomme de guar hydroxypropylée et la gomme de scléroglucane.

18. Utilisation de la composition selon la revendication 17, pour l'obtention d'une coiffure résistant à l'eau.

## Patentansprüche

1. Verfahren zur Formgebung oder Formhaltung der Frisur, bei dem man eine kosmetische Zusammensetzung, die in einem kosmetisch unbedenklichen Nicht-Fett-Medium, dessen Gewichtsmenge an Fettsubstanz zwischen 0 und 10% und vorzugsweise zwischen 0 und 5%, bezogen auf das Gesamtgewicht der Zusammensetzung, liegt, mindestens einen wasserdispergierbaren linearen sulfonierten Polyester und mindestens ein nichtionisches verdickendes Polymer umfasst, verwendet, wobei es sich bei dem mindestens einen wasserdispergierbaren linearen sulfonierten Polyester um ein Polykondensat von mindestens einer Dicarbonsäure oder einem ihrer Ester, mindestens einem Diol und mindestens einer difunktionellen Sulfoaryldicarbonsäureverbindung, die am aromatischen Kern durch eine -SO3M-Gruppe substituiert ist, wobei M für ein Wasserstoffatom oder ein Metallion wie Na+, Li+ oder K+ steht, handelt, wobei das mindestens eine nichtionische verdickende Polymer aus Verdickern auf CelluloseBasis, Guargummi, Hydroxypropylguargummi und Skleroglucangummi ausgewählt ist.

2. Verfahren nach Anspruch 1, **dadurch gekennzeichnet, dass** der wasserdispergierbare lineare sulfonierte Polyester aus Isophthalsäure, Sulfoisophthalsäure-Natriumsalz, Diethylenglykol und 1,4-Cyclohexanmethanol erhalten wird.

3. Verfahren nach einem der vorhergehenden Ansprüche, **dadurch gekennzeichnet, dass** die Tg des wasserdispergierbaren linearen sulfonierten Polyesters bzw. der wasserdispergierbaren linearen sulfonierten Polyester größer gleich 50°C ist.

4. Verfahren nach einem der vorhergehenden Ansprüche, **dadurch gekennzeichnet, dass** die Konzentration an wasserdispergierbarem linearem sulfoniertem Polyester bzw. wasserdispergierbaren linearen sulfonierten Polyestern zwischen 0,1 und 40 Gew.-%, vorzugsweise zwischen 1 und 30 Gew.-% und noch weiter bevorzugt zwischen 5 und 25 Gew.-%, bezogen auf das Gesamtgewicht der Zusammensetzung, liegt.

5. Verfahren nach einem der vorhergehenden Ansprüche, **dadurch gekennzeichnet, dass** die Zusammensetzung mindestens ein nichtionisches verdickendes Polymer umfasst, das aus Cellulosen, die durch Gruppen mit mindestens einer Fettkette modifiziert sind, und Hydroxypropylguargummen, die durch Gruppen mit mindestens einer Fettkette modifiziert sind, ausgewählt ist.

6. Verfahren nach einem der vorhergehenden Ansprüche, **dadurch gekennzeichnet, dass** die Konzentration an nichtionischem verdickendem Polymer bzw. nichtionischen verdickenden Polymeren zwischen 0,01 und 10 Gew.-%, vorzugsweise zwischen 0,05 und 5 Gew.-% und noch weiter bevorzugt zwischen 0,1 und 2 Gew.-%, bezogen auf das Gesamtgewicht der Zusammensetzung, liegt.

7. Verfahren nach einem der vorhergehenden Ansprüche, **dadurch gekennzeichnet, dass** das Verhältnis von wasserdispergierbarem linearem sulfoniertem Polyster zu nichtionischem verdickendem Polymer bzw. nichtionischen verdickenden Polymeren zwischen 2 und 50%, vorzugsweise zwischen 2,5 und 20% und noch weiter bevorzugt zwischen 3 und 15% einschließlich der Grenzwerte liegt.

8. Verfahren nach einem der vorhergehenden Ansprüche, **dadurch gekennzeichnet, dass** die Zusammensetzung ein fixierendes Polymer, das aus anionischen, kationischen, amphoteren, nichtionischen fixierenden Polymeren oder Mischungen davon ausgewählt ist, umfasst.

9. Verfahren nach einem der vorhergehenden Ansprüche **dadurch gekennzeichnet, dass** das kationische fixierende Polymer aus Homopolymeren oder Copolymeren von Acryl- oder Methacrylsäureestern oder -amiden mit Aminfunktionen, kationischen Polysacchariden, quaternären Copolymeren von Vinylpyrrolidon und Vinylimidazol sowie Chitosanen ausgewählt ist.

10. Verfahren nach Anspruch 8, **dadurch gekennzeichnet, dass** das anionische fixierende Polymer aus Homopolymeren oder Copolymeren von Acryl- und Methacrylsäure oder ihren Salzen, Copolymeren von Crotonsäure, Copolymeren von einfach ungesättigten C₄-C₈-Carbonsäuren oder deren Anhydriden, Polyacrylamiden mit Carboxylatgruppen, Homopolymeren oder Copolymeren mit Sulfonsäuregruppen, anionischen Polyurethanen und anionischen Silikonpfropfpolymeren ausgewählt ist.

11. Verfahren nach Anspruch 8, **dadurch gekennzeichnet, dass** das amphotere fixierende Polymer aus Copolymeren mit sauren Vinyleinheiten und mit basischen Vinyleinheiten, vernetzten acylgruppenhaltigen Polyaminoamiden, Polymeren mit zwitterionischen Einheiten, Polymeren, die sich von Chitosan ableiten, modifizierten (C₁-C₅)-Alkylvinylether/Maleinsäureanhydrid-Copolymeren, amphoteren Polyurethanen und amphoteren Silikonpfropfpolymeren ausgewählt ist.

12. Verfahren nach Anspruch 8, **dadurch gekennzeichnet, dass** das nichtionische fixierende Polymer aus Polyalkyloxazolinen, Homopolymeren und Copolymeren von Vinylacetat, Homopolymeren und Copolymeren von Acrylsäureestern, Copolymeren von Acrylnitril, Homopolymeren und Copolymeren von Styrol, Polyamiden, Homopolymeren von Vinyllactam, die von Homopolymeren von Vinylpyrrolidon verschieden sind, Copolymeren von Vinyllactam, nichtionischen Polyurethanen und nichtionischen Silikonpfropfpolymeren ausgewählt ist.

13. Verfahren nach einem der Ansprüche 8 bis 12, **dadurch gekennzeichnet, dass** die Konzentration an fixierendem Polymer bzw. fixierenden Polymeren zwischen 0,1 und 20 Gew.-% und vorzugsweise zwischen 0,5 und 10 Gew.-%, bezogen auf das Gesamtgewicht der Zusammensetzung, liegt.

14. Verfahren nach einem der vorhergehenden Ansprüche, **dadurch gekennzeichnet, dass** die Zusammensetzung ein zusätzliches kationisches, anionisches oder amphoteres verdickendes Polymer umfasst.

15. Verfahren nach dem vorhergehenden Anspruch, **dadurch gekennzeichnet, dass** die Konzentration an zusätzlichem verdickendem Polymer zwischen 0,01 und 20 Gew.-% und vorzugsweise zwischen 0,05 und 10 Gew.-%, bezogen auf das Gesamtgewicht der Zusammensetzung, liegt.

16. Verfahren nach einem der vorhergehenden Ansprüche, **dadurch gekennzeichnet, dass** die Zusammensetzung mindestens ein Additiv, das aus nichtionischen, anionischen, kationischen und amphoteren Tensiden, zusätzlichen nichtionischen, anionischen, kationischen und amphoteren Polymeren, Ceramiden und Pseudoceramiden, Vitaminen und Provitaminen, darunter Panthenol, wasserlöslichen und fettlöslichen, Silikon- und Nichtsilikon-Lichtschutzmitteln, Füllstoffen und festen Teilchen, wie beispielsweise farbigen oder farblosen anorganischen und organischen Pigmenten, Perlglanz- und Trübungsmitteln, Pailletten, Wirkstoffteilchen, Farbmitteln, Sequestriermitteln, Weichmachern, Lösungsvermittlern, Ansäuerungsmitteln, Alkalinisierungsmitteln, Neutralisationsmitteln, anorganischen und organischen Verdickungsmitteln, Antioxidantien, Hydroxysäuren, Penetriermitteln, Duftstoffen und Konservierungsstoffen ausgewählt ist, enthält.

17. Verwendung einer Zusammensetzung, die in einem kosmetisch unbedenklichen Nicht-Fett-Medium, dessen Gewichtsmenge an Fettsubstanz zwischen 0 und 10% und vorzugsweise zwischen 0 und 5%, bezogen auf das Gesamtgewicht der Zusammensetzung, liegt, mindestens einen wasserdispergierbaren linearen sulfonierten Polyester und mindestens ein nichtionisches verdickendes Polymer umfasst, wobei es sich bei dem mindestens einen wasserdispergierbaren linearen sulfonierten Polyester um ein Polykondensat von mindestens einer Dicarbonsäure oder einem ihrer Ester, mindestens einem Diol und mindestens einer difunktionellen Sulfoaryldicarbonsäureverbindung, die am aromatischen Kern durch eine -SO3M-Gruppe substituiert ist, wobei M für ein Wasserstoffatom oder ein Metallion wie Na+, Li+ oder K+ steht, handelt, wobei das mindestens eine nichtionische verdickende Polymer aus Verdickern auf CelluloseBasis, Guargummi, Hydroxypropylguargummi und Skleroglucangummi ausgewählt ist, zur Formgebung oder Formhaltung der Frisur.

18. Verwendung der Zusammensetzung nach Anspruch 17 zum Erhalt einer wasserbeständigen Frisur.

## Claims

1. Process for the shaping or form retention of the hairstyle, in which a cosmetic composition, comprising, in a cosmetically acceptable non-fatty medium, the amount by weight of fatty substance of which is between 0 and 10%, preferably between 0 and 5% relative to the total weight of the composition, at least one water-dispersible linear sulfonic polyester and at least one nonionic thickening polymer, is used, said at least one water-dispersible linear sulfonic polyester being a polycondensate of at least one dicarboxylic acid or one of the esters thereof, of at least one diol and of at least one difunctional sulfoaryldicarboxylic compound substituted on the aromatic ring by a group -SO₃M, in which M represents a hydrogen atom or a metal ion such as Na⁺, Li⁺ or K⁺, said at least one nonionic thickening polymer being chosen from cellulose-based thickeners, guar gum, hydroxypropyl guar gum and scleroglucan gum.

2. Process according to Claim 1, **characterized in that** the water-dispersible linear sulfonic polyester is obtained from isophthalic acid, the sodium salt of sulfoisophthalic acid, diethylene glycol and 1,4-cyclohexanemethanol.

3. Process according to either one of the preceding claims, **characterized in that** the Tg of the water-dispersible linear sulfonic polyester(s) used is greater than or equal to 50°C.

4. Process according to any one of the preceding claims, **characterized in that** the concentration of water-dispersible linear sulfonic polyester(s) is between 0.1% and 40%, preferably between 1% and 30% and even more preferably between 5% and 25% by weight relative to the total weight of the composition.

5. Process according to any one of the preceding claims, **characterized in that** the composition comprises at least one nonionic thickening polymer chosen from:
celluloses modified with groups comprising at least one fatty chain and hydroxypropyl guars modified with groups comprising at least one fatty chain.

6. Process according to any one of the preceding claims, **characterized in that** the concentration of nonionic thickening polymer(s) is between 0.01% and 10%, preferably between 0.05% and 5% and even more preferably between 0.1% and 2% by weight relative to the total weight of the composition.

7. Process according to any one of the preceding claims, **characterized in that** the water-dispersible linear sulfonic polyester/nonionic thickening polymer(s) ratio is between 2% and 50%, preferably between 2.5% and 20% and even more preferentially between 3% and 15%, limits inclusive.

8. Process according to any one of the preceding claims, **characterized in that** the composition comprises a fixing polymer chosen from anionic, cationic, amphoteric and nonionic fixing polymers, or mixtures thereof.

9. Process according to any one of the preceding claims, **characterized in that** the cationic fixing polymer is chosen from homopolymers or copolymers of acrylic or methacrylic esters or amides containing amine functions, cationic polysaccharides, quaternary copolymers of vinylpyrrolidone and of vinylimidazole, and chitosans.

10. Process according to Claim 8, **characterized in that** the anionic fixing polymer is chosen from homopolymers or copolymers of acrylic and methacrylic acid or salts thereof, crotonic acid copolymers, copolymers of C₄-C₈ monounsaturated carboxylic acids or anhydrides. polyacrylamides containing carboxylate groups, homopolymers or copolymers containing sulfonic groups, anionic polyurethanes and anionic grafted silicone polymers.

11. Process according to Claim 8, **characterized in that** the amphoteric fixing polymer is chosen from copolymers containing acidic vinyl units and basic vinyl units, crosslinked and acylated polyamino amides, polymers containing zwitterionic units, chitosan-derived polymers, modified (C₁-C₅) alkyl vinyl ether/maleic anhydride copolymers, amphoteric polyurethanes and amphoteric grafted silicone polymers.

12. Process according to Claim 8, **characterized in that** the nonionic fixing polymer is chosen from polyalkyloxazolines, vinyl acetate homopolymers and copolymers, acrylic ester homopolymers and copolymers, acrylonitrile copolymers, styrene homopolymers and copolymers, polyamides, vinyllactam homopolymers other than vinylpyrrolidone homopolymers, vinyllactam copolymers, nonionic polyurethanes and nonionic grafted silicone polymers.

13. Process according to any one of Claims 8 to 12, **characterized in that** the concentration of fixing polymer(s) is between 0.1% and 20% and preferably between 0.5% and 10% by weight relative to the total weight of the composition.

14. Process according to any one of the preceding claims, **characterized in that** the composition comprises an additional cationic, anionic or amphoteric thickening polymer.

15. Process according to the preceding claim, **characterized in that** the concentration of additional thickening polymer is between 0.01% and 20% and preferably between 0.05% and 10% by weight relative to the total weight of the composition.

16. Process according to any one of the preceding claims, **characterized in that** the composition contains at least one additive chosen from nonionic, anionic, cationic and amphoteric surfactants, nonionic, anionic, cationic and amphoteric additional polymers, ceramides and pseudoceramides, vitamins and provitamins, including panthenol, silicone or non-silicone watersoluble and liposoluble sunscreens, fillers and solid particles, such as, for example, coloured or uncoloured mineral and organic pigments, nacreous agents and opacifiers, flakes, active particles, dyes, sequestering agents, plasticizers, solubilizers, acidifying agents, basifying agents, neutralizers, mineral and organic thickeners, antioxidants, hydroxy acids, penetrants, fragrances and preserving agents.

17. Use, for the fixing and form retention of the hair, of a composition comprising, in a cosmetically acceptable non-fatty medium, the amount by weight of fatty substance of which is between 0 and 10%, preferably between 0 and 5% relative to the total weight of the composition, at least one water-dispersible linear sulfonic polyester and at least one nonionic thickening polymer, said at least one water-dispersible linear sulfonic polyester being a polycondensate of at least one dicarboxylic acid or one of the esters thereof, of at least one diol and of at least one difunctional sulfoaryldicarboxylic compound substituted on the aromatic ring by a group -SO₃M, in which M represents a hydrogen atom or a metal ion such as Na⁺, Li⁺ or K⁺, said at least one nonionic thickening polymer being chosen from cellulose-based thickeners, guar gum, hydroxypropyl guar gum and scleroglucan gum.

18. Use of the composition according to Claim 17, to obtain a water-resistant hairstyle.
